Europäisches Patentamt

(19) European Patent Office

Office européen des brevets

(11) EP 0 452 145 B1

(12) **EUROPEAN PATENT SPECIFICATION**

(45) Date of publication and mention
of the grant of the patent:
**13.11.1996 Bulletin 1996/46**

(51) Int. Cl.⁶: **A61K 9/14**, A61K 9/16,
A61K 9/50, A61K 47/48

(21) Application number: **91303256.1**

(22) Date of filing: **12.04.1991**

(54) **Coated composition and its preparation process**

Ueberzogene Zusammensetzung sowie Verfahren zu ihrer Herstellung

Composition revêtue et son procédé de préparation

(84) Designated Contracting States:
**AT BE CH DE DK ES FR GB GR IT LI LU NL SE**

(30) Priority: **12.04.1990 JP 96663/90**

(43) Date of publication of application:
**16.10.1991 Bulletin 1991/42**

(73) Proprietor: **SHIONOGI SEIYAKU KABUSHIKI KAISHA**
**trading under the name of**
**SHIONOGI & CO. LTD.**
**Osaka 541 (JP)**

(72) Inventors:
• **Nagafuzi, Norboru**
  **Sakai-shi, Osaka-fu (JP)**
• **Tsukada, Takayuki**
  **Itami-shi, Hyogo-ken (JP)**

• **Shima, Kazuhiro**
  **Toyonaka-shi (JP)**
• **Takagishi, Yasushi**
  **Ashiya-shi, Hyogo-ken (JP)**
• **Suzuki, Yusuke**
  **Izumi-shi, Osaka-fu (JP)**
• **Tomoda, Yoshitaka**
  **Osaka-shi, Osaka-fu (JP)**
• **Hayashi, Takashi**
  **Osaka-shi, Osaka-fu (JP)**

(74) Representative: **Hardisty, David Robert et al**
**BOULT, WADE & TENNANT**
**27 Furnival Street**
**London EC4A IPQ (GB)**

(56) References cited:
**EP-A- 0 078 599      EP-A- 0 177 368**
**EP-A- 0 237 345      EP-A- 0 256 127**
**EP-A- 0 368 247      DE-C- 3 721 721**

## Description

The present invention relates to a coated composition and its preparation process. More particularly, it relates to an orally administrable coated composition comprising a pharmaceutically active substance stable to heat, and its preparation process.

For preparation of an orally administerable composition comprising a pharmaceutically active substance, there is commonly adopted a wet granulating and/or coating procedure using a solvent such as water or an organic solvent. When said active substance is sensitive to a solvent, however, such wet procedure is not applicable. In this case, the pharmaceutically active substance or its mixture with any additive such as a filler or a diluent is supplied in the form of a powdery preparation, or is compressed and granulated to make a granular preparation. The powdery or granular preparation is, when desired, filled in gelatin capsules to make a capsule preparation or compressed to make a tablet preparation. Since these preparations are uncoated, they may afford an unpleasant taste to patients who take them. Further, such a lack of coating makes it difficult to control the site or time at which the pharmaceutically active substance exerts its pharmaceutical efficacy.

EP-A-0 256 127 discloses (see Example 1) a coated composition comprising granules comprising a pharmaceutically active substance (a protease) cohering with each other with the aid of a first thermomelting material ("PEG 5000", mp.58°C) each of said granules being coated with a second thermomelting material ("PEG 1540", mp.43°C). The granules are being prepared in a granulating machine (250 rpm) under heating without the use of any solvent.

In order to coat a solvent sensitive pharmaceutically active substance, an extensive study has been made, and as a result, there is now completed a new process for preparing a coated composition comprising a pharmaceutically active substance without using any solvent.

According to this invention there is provided an orally administrable coated composition comprising a pharmaceutically active substance stable to heat, which comprises granules comprising fine particles of said active substance cohering each other by the aid of a first thermomelting material as a binder, each of said granules being coated with a second thermomelting material having a melting point lower than that of the first thermomelting material in an amount of from about 0.1 to 2 parts by weight to one part by weight of said granule, said granules being prepared by a centrifuged force granulation procedure without using any solvent, and a process for the preparation of said composition.

In view of the characteristic nature of the process, the pharmaceutically active substance is required to be the one which is stable to heat, for instance, substantially stable at a temperature not exceeding about 200°C. The process is advantageously applicable, particularly when said active substance is sensitive to a solvent such as water or an organic solvent, because it does not require any solvent. The term "sensitive" used herein means that the pharmaceutically active substance is readily (e.g. within about one hour) influenced by a solvent to such an extent that its physical, chemical and/or biological properties are substantially modified or changed. Specific examples of the pharmaceutically active substances are antihistamines including terfenadine, chlorpheniramine maleate, clemastine fumarate, carbinoxamine maleate, promethazine hydrochloride, and diphenhydramine salycylate; analgesics and antipyretics including aspirine, salicylamide, ethanzamide, acetaminophen and diclofenac sodium; bronchodilators including S-1452 and cyclophosphamide; antitussives and expectorants including dextromethorphan hydrobromide, dihydrocodeine phosphate, cloperastine hydrochloride, phenylpropanolamine hydrochloride, methylephedrine, potassium cresol sulfonate, morphine sulphate, codeine phosphate and belladonna total alkaloids; antiulcers including benexate hydrochloride betadex (hereinafter referred to as "TA 903"), pirenzepine, cetraxate, ranitidine and famotidine; drugs for circulatory organs including pindolol, propranolol, alprenolol, oxprenolol, diltiazem and pinacidil; antitumors including 5-fluorouracil and tegafur; antibiotics including cefalexin, cefaclor and S-1108; antibacterials including cinoxacin, enoxacin and lomefloxacin, etc.

The thermomelting material (covering the first thermomelting material and the second thermomelting material) may be the one which readily melts at an elevated temperature such as 40°C or higher, preferably from 50 to 150°C, to give a melt having a low viscosity, preferably of such an extent as showing a self-flowability or self-fluidity. Typical examples of such thermomelting materials are polyethylene glycols.

The thermomelting material may comprise a single hydrophilic or hydrophobic material or a mixture comprising at least one hydrophilic material and at least one hydrophobic material. The use of a mixture is sometimes recommendable to make the melting point lower. Also, the use of such mixture makes it possible to control the time for breaking or dissolving the coated composition, i.e. coated granules, as the final product in digestive organs.

Examples of the hydrophilic material are polyethylene glycols (PEG: macrogol) having an average molecular weight of not less than about 400, preferably of not less than about 1,000, more preferably from about 2,000 to 20,000, saccharides (e.g. D-glucose, maltose, fructose), sugar alcohols (e.g. D-mannitol, D-sorbitol), surfactants (e.g. sorbitan, Pluronic F68), etc. Among them, polyethylene glycols are usually preferred. Examples of hydrophobic materials are straight-chain saturated hydrocarbons (e.g. vaseline, paraffin), fats and oils (e.g. cacao butter, beef tallow, lard, hydrogenated soybean oil, hydrogenated castor oil), animal and plant waxes (e.g. yellow beeswax, white beeswax), higher fatty acids (e.g. stearic acid), higher alcohols (e.g. cetanol, stearyl alcohol), hydrogenated plant oils (e.g. hydrogenated castor oil (Lubriwax 101), hydrogenated rapeseed oil (Lubriwax 103), polishing wax (a mixture of carnauba wax and paraffin), Presirol (a mixture of glycerol mono-, di- and tripalmitates)), etc.

One or more thermomelting materials appropriately chosen from hydrophilic and hydrophobic materials as above mentioned may be used as the binder (i.e. the first thermomelting material) at the granulating step and as the coating material (i.e. the second thermomelting material) at the coating step. The second thermomelting material to be used as the coating material has a lower melting point than that of the first thermomelting material to be used as the binder in order to prevent the softening or deformation of the granules as prepared in the granulating step. When, for instance, polyethylene glycol 6,000 is used as the binder at the granulating step, the use of polyethylene glycol 4,000 as the coating material at the coating step is favorable. Alternatively, a single thermomelting material may be used as the binder at the granulating step, and its mixture with any other thermomelting material(s) may be used as the coating material at the coating step, because a mixture of two or more thermomelting materials shows a lower melting point than that of each of them.

As the binder at the granulating step, the use of polyethylene glycol 6000 or hydrogenated castor oil is usually preferred. As the coating material at the coating step, the use of a mixture comprising a hydrophilic material and a hydrophobic material respectively in amounts of 5 to 30 % by weight and 10 to 60 % by weight is favorable.

If desired, the thermomelting material as the binder or the coating material may comprise any additive. When, for instance, such a thermomelting material is apt to be auto-oxidized (e.g. polyethylene glycol, triglycerides) is used as the binder, an anti-oxidizing agent, particularly in an oily state at room temperature (e.g. alpha-dl-tocopherol, alpha-d-tocopherol, alpha-d-tocopherol acetate) may be incorporated therein in a small amount such as 100 to 5,000 ppm, preferably 500 to 2,000 ppm, for prevention of the auto-oxidation. Such addition of an anti-oxidizing agent sometimes produces a stabilization effect on the pharmaceutically active substance.

Further, for instance, additives as conventionally employed in solid pharmaceutical preparations may be incorporated into a thermomelting material as the coating material so as to control (i.e. promote or delay) disintegration or dissolution of the coated composition, i.e. coated granules, as the final product. Examples of such additives are saccharides (e.g. lactose), starchs (e.g. wheat starch, corn starch), inorganic materials (e.g. talc, calcium carbonate, titanium dioxide), etc.

Furthermore, for instance, conventional auxiliary agents such as pigments, flavors, stabilizers, preservatives and buffers may be used on preparation of the granules or the coated granules.

The binder may be used normally in such an amount as from 0.05 to 0.4 parts by weight to one part by weight of the pharmaceutically active substance. The coating material may be used usually in such an amount as from 0.1 to 2 parts by weight to one part by weight of the granules. The coating layer to be formed on the granules is not necessarily required to be single and may have two or more layers. For instance, a single coating layer may be formed by the use of a coating material comprising a hydrophilic thermomelting material and a hydrophobic thermomelting material, or two coating layers may be formed by the use of a hydrophilic thermomelting material first and then by the use of a hydrophobic thermomelting material. A typical example is the formation of a single coating layer or two coating layers by the use of 0.05 to 0.3 parts by weight, preferably 0.08 to 0.2 parts by weight, of a hydrophilic thermomelting material and 0.1 to 0.6 parts by weight, preferably 0.2 to 0.4 part by weight of a hydrophobic thermomelting material based on 1 part by weight of the granules.

As stated above, the process of the invention comprises the steps of granulating and coating. Granulating is usually achieved by a centrifuged force granulating procedure, particularly a centrifuged force powder coating procedure. On the other hand, coating nay be attained by various procedures such as a coating procedure using a pan or a coating procedure using a fluidized layer. As a typical example, granulating is carried out by rotating a mixture comprising the pharmaceutically active substance and a first thermomelting material as the binder under conditions such that the first thermomelting material is kept in a melt state, and coating is carried out by rotating the resulting granules while application of a second thermomelting material as the coating material thereto under conditions such that the second thermomelting material is kept in a melt state.

The steps of granulating and coating will be hereinafter explained in more detail.

The granulating step is normally effected by the use of a mixer (i.e. granulator) of rotary type or rolling type (e.g. universal mixer, high-speed mixer, super-mixer, centrifugal granulator) equiped with a heat cotrol apparatus such as a jacket for circulating a heat-exchange fluid or a ventilator for heating and cooling. The container (i.e. a chamber wherein the starting materials are charged and granules are prepared) of the mixer is pre-heated to a temperature higher, preferably 20 - 40°C higher, than the melting point of a first thermomelting material as the binder. Into the container thus pre-heated, a powdery premix of the pharmaceutically active substance and the first thermomelting material is charged, and rotation (preferably with agitation) is effected, whereby the first thermomelting material forms a melt and the particles of the pharmaceutically active substance cohere by the aid of the melt to give granules. Alternatively, the powdery premix may be charged into the container without its pre-heating, followed by heating to fuse the first themomelting material and rotation to make the granules of the pharmaceutically active substance. Once the granulation begins, the granules start to roll, and their particle size grows with application of the powdery premix thereto under rotation to give a desired particle size.

In order to enhance the efficiency of the granulation, nuclear or core particles such as microcrystalline cellulose (e.g. "Avicel" manufactured by Asahi Chemical) or granulated sugar may be introduced into the container of the mixer prior to the charge of the powdery premix. The container is heated while rotating, and then the powdery premix is charged therein, whereby granulation starts. Rotation of the granules is continued with portionwise addition of the powdery premix thereto until the granules grow to a desired particle size.

After growth of the granules to a desired particle size, the temperature of the container is lowered to 40°C or less so that the granules are cooled to give hard and compact particles.

The above prepared granules, i.e. non-coated granules, are then subjected to coating to obtain coated granules. While the coating may be accomplished by various procedures, it is preferable to carry out such coating using a mixer of the same kind as employed in the granulating step. For the sake of convenience, the same mixer as actually used in the granulating step may be employed in the coating step.

Thus, the container of the mixer is pre-heated to keep at a temperature higher, usually about 20°C higher, than the melting point of a second thermomelting material as a coating material. Into the container thus pre-heated, the non-coated granules as obtained in the granulating step are charged, and rotation is carried out. Under continuing rotation, the second thermomelting material or its mixture with any additive in a powdery state is portionwise added thereto, whereby a coating layer is formed on the surface of each particles. The thickness of the coating layer is appropriately controlled with the amount of the coating material, and as the result, the site or time for disintegration or dissolution of the coated granules as the final product can be properly regulated. In the above operation, the coating material comprising a second thermomelting material optionally with an additive may be charged first in the container without pre-heating and then subjected to heating under rotation until a coating layer having a desired thickness is formed on each particle. The formation of the coating layer may be effected in two or more stages using a coating material(s) of the same or different composition(s).

When the coating layer reaches to a desired thickness, the temperature of the container is lowered, for instance, to 40°C or less for cooling under rotation so that coated granules having hard and compact coating layers at the surfaces are obtained.

When desired, any other appropriate operation may be applied between the granulating step and the coating step as above. For instance, the formation of a coating film comprising talc on the surfaces of the non-coated granules may be effected after the granulating step and before the coating step. Such talc film coating is effective in enhancing the physical strength of the non-coated granules so that those granules are protected from breakage or disintegration during coating. It is also effective in taking up the pharmaceutically active substance in a powder form completely so that its attachment onto the surfaces of the granules in the coating step can be avoided. Incorporation of a suitable disintegrating agent into talc is sometimes advantageous, because the release of the pharmaceutically active substance from the resulting granules is delayed for a certain period of time. For the film formation, talc may be used in an amount of 1 to 30 % by weight, preferably of 5 to 20 % by weight based on the weight of the granules.

Practical embodiments of the invention are illustratively shown in the following Examples wherein % and part(s) are by weight unless otherwise indicated.

Example 1 (Granulating)

Benaxate hydrochloride/beta-cyclodextrin inclusion compound (i.e. TA 903) (40 parts), powdery polyethylene glyclol 6000 (i.e. PEG 6000) having a particle size of 300 $\mu$m (48 mesh) or less (18 parts) and sugar powder (4 parts) were mixed together to make a uniform mixture. The mixture (150 g) was charged into an agitation type mixer ("Super-mixer" Type SM-5, manufactured by Kawata Seisakusho), and hot water was circulated through the jacket of the mixer, whereby the container of the mixer was heated to about 95 to 100°C. The agitation blade started rotating slowly, and when PEG 6000 was melted to wet the entire mixture, the speed of rotation was raised to about 900 rpm so that the granulation started to give fine spherical particles. The spherical partices were rolled in the container, during which a mixture having the same composition as above (350 g) was portionwise added thereto so as to make the spherical particles grow. Water was introduced into the jacket of the mixer so that the container was cooled to a temperature below 40°C.

The thus obtained granules had the following size distribution:

| Particle size ($\mu$m) | Amount (g) |
|---|---|
| more than 710 (24 mesh on) | 22.2 (4.5 %) |
| 710 - 420 (24 - 35 mesh) | 149.7 (30.4 %) |
| 420 - 150 (35 - 100 mesh) | 302.0 (61.4 %) |
| less than 150 (100 mesh pass) | 18.1 (3.7 %) |
| Total | 492.0 |

Example 2 (Granulating)

Granules having a particle size of 150 to 420 $\mu$m (35 to 100 mesh) (150 g) as obtained in Example 1 were charged in the container of a mixer, and the agitation blade was rotated slowly. The temperature of the container was elevated to about 95 to 100°C with slow rolling of the granules. When the surfaces of the granules began to melt, the rotation speed was raised, and a mixture having the same composition as used in Example 1 (350 g) was portionwise added thereto so as to make the spherical particles grow. Water was introduced into the jacket of the mixer so that the container was cooled to a temperature below 40°C.

The thus obtained granules had the following size distribution:

| Particle size ($\mu$m) | Amount (g) |
|---|---|
| more than 710 (24 mesh on) | 14.6 (3.0 %) |
| 710 - 420 (24 - 35 mesh) | 323.3 (65.5 %) |
| 420 - 150 (35 - 100 mesh) | 103.9 (21.1 %) |
| less than 150 (100 mesh pass) | 51.5 (10.4 %) |
| Total | 493.3 |

Example 3 (Coating)

Stearyl alcohol (6 parts) and polyethylene glycol 4000 (i.e. PEG 4000) (4 parts) were mixed together, and the resultant mixture was heated at 80°C for fusion, followed by agitation. After cooling, the solidified product was crushed and pulverized to make a premix having a particle size of 300 $\mu$m or less as a coating material. Using the premix, the following two coating compositions were prepared:

| First coating composition: | |
|---|---|
| | Part(s) |
| Talc | 2 |
| Corn starch | 2 |
| Premix | 1 |
| Total | 5 |

| Second coating composition: | |
|---|---|
| | Part(s) |
| Talc | 2 |
| Lubriwax | 2 |
| Premix | 1 |
| Total | $\overline{5}$ |

The granules having a particle size of 420 to 710 $\mu$m (24 to 35 mesh) (250 g) as prepared in Example 1 or 2 were charged into the container of a mixer ("Super-mixer" Type SM-5) and rotated slowly under agitation, whereby the granules were rolled. Hot water was circulated in the jacket of the mixer, and the temperature of the container was elevated to about 60°C. Then, the rotation speed was raised, and the first coating composition (150 g) was portionwise added thereto so as to coat the granules. Then, water was introduced into the jacket of the mixer to lower the temperature of the container below 40°C.

The coated granules thus obtained had a particle size of 420 to 1,000 $\mu$m (16 to 35 mesh) and a total weight of 402.5 g. The coating rate as calculated according to the following formula was 61 %:

$$\frac{\text{Weight of granules after coating - Weight of granules before coating}}{\text{Weight of granules before coating}} \times 100$$

Hot water was again introduced into the jacket so as to elevate the temperature of the container to about 57°C. The above obtained coated granules (402 g) were charged into the container, and rotation with agitation was carried out, during which the second coating composition was portionwise added thereto to coat the surfaces of the coated granules. Water was introduced into the jacket so as to lower the temperature of the container below 40°C.

The coated granules thus obtained had a particle size of 420 to 1,000 $\mu$m (16 to 35 mesh) and a total weight of 482 g. The coating rate as calculated according to the following formula was 20 %:

$$\frac{\text{Weight of the second coated granules - Weight of the first coated granules}}{\text{Weight of the first coated granules}} \times 100$$

Example 4 (Coating)

The premix as prepared in Example 3 (1 part) was mixed together with talc (2 parts), corn starch (1.2 parts) and Lubriwax 101 (0.8 part) to give a powdery mixture for delayed release coating. The granules having a particle size of 420 to 710 $\mu$m (24 to 35 mesh) (250 g) as prepared in Example 1 or 2 were charged into the container of a mixer ("Super-mixer" Type SM-5), and slow rotation with agitation was carried out, during which hot water was circulated through the jacket of the mixer to heat the container to a temperature of about 58°C. The rotation speed was raised, and the powdery mixture was portionwise added thereto to make a coating layer at the surfaces of the granules. Then, make a coating layer at the surfaces of the granules. Then, water was introduced into the jacket to lower the temperature of the container below 40°C, and the coated granules were cooled.

The coated granules thus obtained had a particle size of 420 to 1,000 $\mu$m (16 to 35 mesh) and a total weight of 510 g. The coating rate as calculated according to the formula given in Example 3 was 104 %.

Example 5 (Granulating with cores)

TA 903 pulverized to a particle size of 150 $\mu$m or less (100 mesh pass) (20 parts), PEG 6,000 (9 parts) and sugar powder (2 parts) were mixed uniformy to make a premix for granulation. Granular sugar having a particle size of 210 to 300 $\mu$m (48 to 65 mesh) (150 g) as nuclear or core particles was charged in the container of a mixer ("Super-mixer" Type SM-5) and slowly rotated with agitation while heating to about 95°C or more by circulating hot water through the jacket. After the nuclear or core particles were heated, the rotation speed was raised to about 900 rpm, and the premix (400 g) was portionwise added thereto. Water was introduced into the jacket, and the container was cooled to a temperature below 40°C.

The thus obtained granules had the following size distribution:

| Particle size (μm) | Amount (g) |
|---|---|
| more than 840 (20 mesh on) | 56.1 (10.3 %) |
| 840 - 500 (20 - 32 mesh) | 76.0 (14.0 %) |
| 500 - 300 (32 - 48 mesh) | 333.0 (61.2 %) |
| 300 - 210 (48 - 65 mesh) | 70.0 (12.9 %) |
| less than 200 (65 mesh pass) | 9.1 (1.6 %) |
| Total | 544.2 |

Example 6 (Granulating with cores)

PEG 6000 having a particle size of about 180 μm, or less (80 mesh pass) was admixed with alpha-d-tocopherol (i.e. VE) in an amount of 900 ppm to make a uniform mixture (i.e. VE-added PEG 6000). VE-added PEG 6000 (15 kg) and TA 903 powder (40 kg) were mixed together and passed through a screen (80 mesh) to make a premix for granulation. Separately, talc (9.5 kg) was admixed with VE-added PEG 6000 (250 g) and microcrystalline cellulose (1.25 kg) as a disintegrating agent to make a mixed powder for talc film formation.

Into the container of a centrifugal rotary granulator ("CF-1000" manufactured by Freund Industry) provided with a regulator for heating temperature, the wall temperature of said container having been adjusted to about 106°C, microcrystalline cellulose powder "Avicel") having a 50% average particle size of 350 μm (27.57 kg) as nuclear or core particles was charged, and rotation was carried out at 100 rpm. When the core or nuclear particles were heated to about 95°C, the above prepared premix for granulation was portionwise applied thereto with a supply speed of 0.5 kg/min. With the progress of the fusion of PEG 6000 in the premix, the nuclear or core particles were wetted, and the amount of TA 903 powder attaching to the nuclear or core particles was increased. The supply speed of the premix was gradually increased until the attaching rate of TA 903 powder was made constant, and then the remaining amount of the premix was applied thereto with a supply speed of 3 kg/min to make hard and compact spherical granules. In comparison with the weight of the nuclear or core particles, the weight increase was 199.5 %.

Onto the spherical granules thus obtained, an entire amount of the above prepared mixed powder for talc coating was applied at a supply speed of 3 kg/min, followed by cooling to about 70°C to make a talc coating film at the surfaces of the spherical granules. In comparison with the weight of the spherical granules, the weight increase was 13.3 %.

The thus obtained granules having a talc coating film on the surfaces had an average particle size of about 600 μm and an average particle weight of about 938 (containing about 400 mg of TA 903). Their particle size distribution was as follows:

| Particle size (μm) | Amount (kg) |
|---|---|
| more than 840 (24 mesh on) | 4.10 (4.3 %) |
| 840 - 355 (20 - 42 mesh) | 87.38 (93.2 %) |
| less than 355 (42 mesh pass) | 0.16 (0.2 %) |
| Total | 91.64 |

Of the granules having a talc coating film on the surfaces as above prepared, those having an average particle size of 355 to 840 μm (20 to 42 mesh) were collected and subjected to coating in Example 7.

Example 7 (Coating)

PEG 4000 having a particle size of about 180 μm or less (80 mesh pass) was admixed with VE in an amount of 900 ppm to make a uniform mixture (i.e. VE-added PEG 4000). VE-added PEG 4000 (3.94 kg), stearyl alcohol powder having a particle size of about 180 μm or less (80 mesh pass) (2.64 kg), corn starch (2.64 kg), hydrogenated castor oil (7.88 kg) and talc (13.93 kg) were mixed together to make a mixed powder for coating.

Into the container of the granulator as used in Example 6, said container having been pre-heated to about 65°C, the granules having a talc coating film on the surfaces (355 to 840 μm) (50.00 kg) as prepared in Example 6 were charged, and rotation was carried out at 100 rpm. When the temperature of the granules reached a temperature of about 62°C, the mixed powder for coating as above prepared was portionwise applied thereto with a supply speed of 0.2 kg/min. With the progress of the fusion of PEG 4000 in the mixed powder, the granules were wetted, and the amount of the mixed powder attaching to the surfaces of the granules was increased. The supply speed of the mixed powder was gradually increased until the attaching rate of the mixed powder was made constant, and then the remaining amount of the mixed powder was applied thereto with a supply speed of 3 kg/min, followed by cooling the temperature below 50°C to make the hard and compact coated granules. In comparison with the weight of the granules, the weight increase was 56.8 %.

The thus obtained coated particles had an average particle size of about 700 μm and an average particle weight 1541 mg (containing 400 mg of TA 903). Their particle size distribution was as follows:

| Particle size (μm) | Amount (kg) |
|---|---|
| more than 1000 (16 mesh on) | 0.37 (0.4 %) |
| 1000 - 355 (16 - 42 mesh) | 83.03 (98.7 %) |
| less than 355 (42 mesh pass) | 0.51 (0.6 %) |
| Total | 83.91 |

Test Example 1

With the coated granules having two coating layers at the surfaces (particle size, 420 to 1000 μm (16 - 35 mesh)) as obtained in Example 3, the dissolution test was carried out according to the puddle method as described in Japanese Pharmacopoeia XI. As the dissolution media, there were used the 1st test solution (prepared by adding water to a mixture of sodium chloride (2.0 g) and dilute hydrochloric acid (24.0 ml) to make a total volume of 1,000 ml, pH, about 1.2) (900ml) and purified water (900 ml). Measurement was made on TA 903 under a rotation speed of 100 rpm.

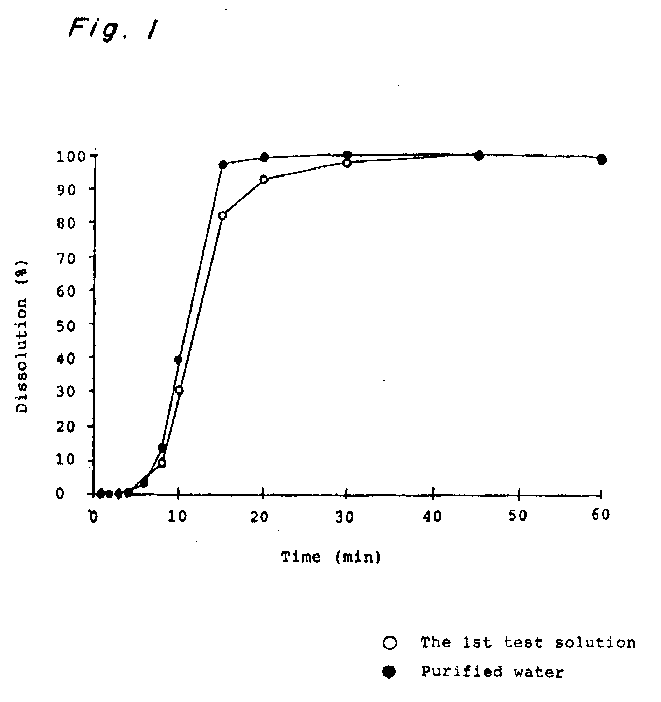

The results (dissolution curve) are shown in Fig. 1 of the accompanying drawings, from which it is understood that the dissolution of TA 903 is delayed about 5 minutes. In fact, the release of TA 903 from the coated granules as above in the mouth was prevented for several minutes, and therefore no bitter taste was produced on the oral administration.

Test Example 2

With the coated granules (particle size, 420 to 1000 μm (16 - 35 mesh)) as obtained in Example 4, the disolution test was carried out according to the puddle method as described in Japanese Pharmacopoeia XI. As the disolution media, there were used the 1st test solution (900 ml) and purified water (900 ml). Measurement was made on TA 903 under a rotation speed of 100 rpm.

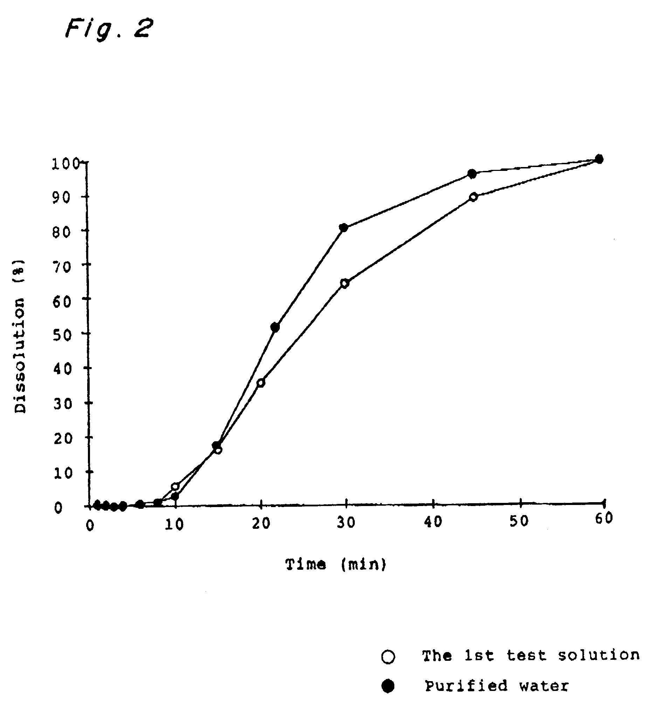

The results (dissolution curve) are shown in Fig. 2 of the accompanying drawings, from which it is understood that the dissolution of TA 903 is delayed about 5 minutes, and then performed gradually. Such dissolution curve indicates the characteristics of the coated granules of a release controlled type.

Test Example 3

With the coated granules (average particle size, about 700 μm) as obtained in Example 7, the dissolution test was carried out according to the puddle method as described in Japanese Pharmacopoeia XI. As the dissolution media, there were used the 1st test solution (900 ml), the 2nd test solution (prepared by adding water to a mixture of 0.2 M potassium dihydrogen phosphate solution (250 ml) and 0.2 N sodium hydroxide solution (118 ml) to make a total volume of 1,000 ml; pH, about 6.8) (900 ml) and purified water (900 ml). Measurement was made on TA 903 under a rotation speed of 100 rpm.

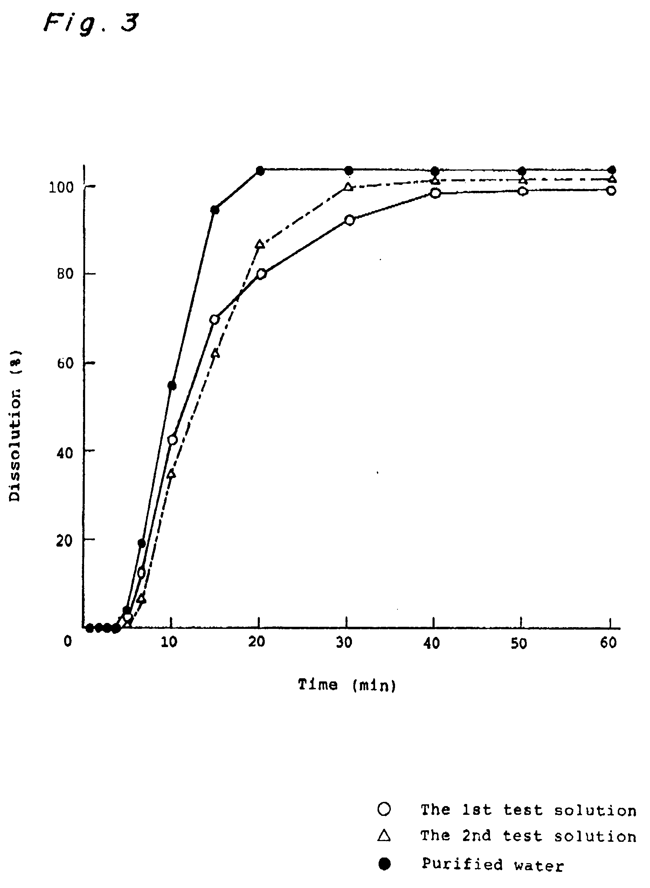

The results (dissolution curve) are shown in Fig. 3 of the accompanying drawings, from which it is understood that the dissolution of TA 903 is delayed about 5 minutes.

**Claims**

**Claims for the following Contracting States : AT, BE, CH, DE, DK, FR, GB, IT, LI, LU, NL, SE**

1. An orally administrable coated composition comprising a pharmaceutically active substance stable to heat, which comprises granules comprising fine particles of said active substance cohering to each other by means of a first thermomelting material as a binder, each of said granules being coated with a second thermomelting material having a melting point lower than that of the first thermomelting material in an amount of from 0.1 to 2 parts by weight to one part by weight of said granule, said granules being prepared by a centrifuged force granulation procedure without using any solvent.

2. The coated composition according to claim 1, wherein the granules do not substantially release said pharmaceutically active substance for at least 5 minutes after the oral administration.

3. The coated composition according to claim 1 or claim 2, wherein the first thermomelting material is polyethylene glycol which may comprise a small amount of alpha-d-tocopherol.

4. The coated composition according to claim 3 wherein the polyethylene glycol has an average molecular weight of not less than about 1000.

5. The coated composition according to any one of the preceding claims wherein the coating is formed in a bi-layer which comprises an inner layer comprising a hydrophilic material and an outer layer comprising a hydrophobic material.

6. The coated composition according to any one of the preceding claims, wherein the active substance is a cyclodextrin inclusion compound of 4-guanidinomethylcyclohexanecarboxylic acid 2'-benzyloxycarbonylphenyl ester hydrochloride.

7. A process for preparing a coated composition claimed in any one of the preceding claims, which comprises centrifugally granulating a mixture comprising said active substance and a first thermomelting material as a binder under heating without using any solvent and coating the resultant granules with a second thermomelting material as a coating material under heating, the melting point of the second thermomelting material being lower than that of the first thermomelting material.

8. A process according to claim 7 wherein the granulating is carried out by rotating the mixture under conditions such that the first thermomelting material is kept in a melt state.

9. A process according to claim 7 or claim 8 wherein the coating is carried out by rotating the granules while applying the second thermomelting material thereto under conditions such that the second thermomelting material is kept in a melt state.

10. A process according to any one of claims 7 to 9 wherein the second thermomelting material is a mixture comprising a hydrophilic material and a hydrophobic material in amounts of from 5% to 30% by weight and 10% to 60% by weight respectively based on the mixture.

**Claims for the following Contracting States : ES, GR**

1. A process for preparing an orally administrable coated composition comprising a pharmaceutically active substance stable to heat, which comprises granules comprising fine particles of said active substance cohering to each other by means of a first thermomelting material as a binder, each of said granules being coated with a second thermomelting material having a melting point lower than that of the first thermomelting material in an amount of from 0.1 to 2 parts by weight to one part by weight of said granules, which method comprises centrifugally granulating a mixture comprising said active substance and a first thermomelting material as a binder under heating without using any solvent and coating the resultant granules with a second thermomelting material as a coating material under heating.

2. A process according to claim 1 wherein the granulating is carried out by rotating the mixture under conditions such that the first thermomelting material is kept in a melt state.

3.  A process according to claim 1 or claim 2 wherein the coating is carried out by rotating the granules while applying the second thermomelting material thereto under conditions such that the second thermomelting material is kept in a melt state.

4.  A process according to any one of the preceding claims wherein the second thermomelting material is a mixture comprising a hydrophilic material and a hydrophobic material in amounts of from 5% to 30% by weight and 10% to 60% by weight respectively based on the mixture.

5.  A process according to any of the preceding claims wherein the granules do not substantially release said pharmaceutically active substance for at least 5 minutes after the oral administration.

6.  A process according to any one of the preceding claims wherein the first thermomelting material is polyethylene glycol which may comprise a small amount of alpha-d-tocopherol.

7.  A process according to claim 6 wherein the polyethylene glycol has an average molecular weight of not less than about 1000.

8.  A process according to any one of the preceding claims wherein the coating is formed in a bi-layer which comprises an inner layer comprising a hydrophilic material and an outer layer comprising a hydrophobic layer.

9.  A process according to any one of the preceding claims wherein the active substance is a cyclodextrin inclusion compound of 4-guanidinomethylcyclohexanecarboxylic acid 2'-benzyloxycarbonylphenyl ester hydrochloride.

**Patentansprüche**

**Patentansprüche für folgende Vertragsstaaten : AT, BE, CH, DE, DK, FR, GB, IT, LI, LU, NL, SE**

1.  Oral verabreichbare beschichtete Zusammensetzung beinhaltend einen pharmazeutischen, hitzebeständigen Wirkstoff, die Körner mit feinen Teilchen des besagten Wirkstoffes enthält, wobei die Teilchen mittels eines ersten thermoschmelzenden Materials als Bindemittel aneinander haften, wobei jedes der besagten Körner mit einem zweiten thermoschmelzenden Material, dessen Schmelzpunkt tiefer liegt als derjenige des ersten thermoschmelzenden Materials, in einer Menge von 0,1 bis 2 Gewichtsteilen pro Gewichtsteil des besagten Korns beschichtet ist, und wobei die besagten Körner ohne Verwendung eines Lösungsmittels durch ein Zentrifugalkraft-Granulierverfahren hergestellt werden.

2.  Beschichtete Zusammensetzung nach Anspruch 1, wobei die Körner während mindestens 5 Minuten nach der oralen Einnahme den besagten pharmazeutischen Wirkstoff nicht wesentlich freisetzen.

3.  Beschichtete Zusammensetzung nach Anspruch 1 oder 2, wobei das erste thermoschmelzende Material Polyethylenglykol ist, das eine kleine Menge von alpha-d-Tocopherol enthalten kann.

4.  Beschichtete Zusammensetzung nach Anspruch 3, wobei das Polyethylenglykol ein mittleres Molekulargewicht von nicht weniger als 1000 aufweist.

5.  Beschichtete Zusammensetzung nach irgend einem der vorangehenden Ansprüche, wobei die Beschichtung als Doppelschicht ausgebildet ist, die eine innere Lage, enthaltend einen hydrophilen Stoff, und eine äussere Lage, enthaltend einen hydrophoben Stoff, umfasst.

6.  Beschichtete Zusammensetzung nach irgend einem der vorangehenden Ansprüche, wobei der Wirkstoff eine Cyclodextrin-Einschlussverbindung des Hydrochlorids von 4-Guanidino-methylcyclohexancarbonsäure (2'-benzyloxycarbonyl)phenylester ist.

7.  Verfahren zur Herstellung der beschichteten Zusammensetzung nach irgend einem der vorangehenden Ansprüche, bei dem eine Mischung, die den besagten Wirkstoff und ein erstes thermoschmelzendes Material als Bindemittel enthält, mittels Zentrifugalkraft und ohne Verwendung eines Lösungsmittels granuliert wird und die erhaltenen Körner mit einem zweiten thermoschmelzenden Material als Beschichtung unter Erhitzen beschichtet werden, wobei der Schmelzpunkt des zweiten thermoschmelzenden Materials tiefer liegt als derjenige des ersten thermoschmelzenden Materials.

8. Verfahren nach Anspruch 7, wobei die Granulierung durchgeführt wird, indem die Mischung unter Bedingungen rotiert wird, bei denen das erste thermoschmelzende Material in einem geschmolzenen Zustand gehalten wird.

9. Verfahren nach Anspruch 7 oder 8, wobei die Beschichtung durchgeführt wird, indem die Körner während des Auftragens des zweiten thermoschmelzenden Materials unter Bedingungen rotiert werden, bei denen das zweite thermoschmelzende Material in geschmolzenem Zustand gehalten wird.

10. Verfahren gemäss irgend einem der Ansprüche 7 bis 9, wobei das zweite thermoschmelzende Material eine Mischung ist, die einen hydrophilen Stoff und einen hydrophoben Stoff in Mengen von 5 bis 30 Gewichtsprozenten beziehungsweise 10 bis 60 Gewichtsprozenten, bezogen auf die Mischung, enthält.

**Patentansprüche für folgende Vertragsstaaten : ES, GR**

1. Verfahren zur Herstellung einer oral verabreichbaren beschichteten Zusammensetzung, beinhaltend einer, pharmazeutischen, hitzebeständigen Wirkstoff, die Körner mit feinen Teilchen des besagten Wirkstoffes enthält, wobei die Teilchen mittels eines ersten thermoschmelzenden Materials als Bindemittel aneinander haften, wobei jedes der besagten Körner mit einem zweiten thermoschmelzenden Material, dessen Schmelzpunkt tiefer liegt als derjenige des ersten thermoschmelzenden Materials, in einer Menge von 0,1 bis 2 Gewichtsteilen pro Gewichtsteil der besagten Körner beschichtet ist, wobei dieses Verfahren das zentrifugale Granulieren einer Mischung, die den besagten Wirkstoff und ein erstes thermoschmelzendes Material als Bindemittel beinhaltet, unter Erhitzen und ohne Verwendung irgend eines Lösungsmittels sowie das Beschichten, unter Erhitzen, der erhaltenen Körner mit einem zweiten thermoschmelzenden Material als Beschichtungsmaterial umfasst.

2. Verfahren nach Anspruch 1, wobei das Granulieren durchgeführt wird, indem die Mischung unter Bedingungen rotiert wird, bei denen das erste thermoschmelzende Material in einem geschmolzenen Zustand gehalten wird.

3. Verfahren nach Anspruch 1 oder 2, wobei das Granlieren durchgeführt wird, indem die Körner unter gleichzeitigem Auftragen des zweiten thermoschmelzenden Materials unter Bedingungen rotiert werden, bei denen das zweite thermoschmelzende Material in einem geschmolzenen Zustand gehalten wird.

4. Verfahren nach irgend einem der vorangehenden Ansprüche, wobei das zweite thermoschmelzende Material eine Mischung ist, die ein hydrophiles Material und ein hydrophobes Material in Mengen von 5 bis 30 Gewichtsprozenten beziehungsweise 10 bis 60 Gewichtsprozenten, bezogen auf die Mischung, enthält.

5. Verfahren nach irgend einem der vorangehenden Ansprüche, wobei die Körner während mindestens 5 Minuten nach der oralen Einnahme den besagten pharmazeutischen Wirkstoff nicht wesentlich freisetzen.

6. Verfahren nach irgend einem der vorangehenden Ansprüche, wobei das erste thermoschmelzende Material Polyethylenglykol ist, das eine kleine Menge man alpha-d-Tocopherol enthalten kann.

7. Verfahren nach Anspruch 6, wobei das Polyethylenglykol ein mittleres Molekulargewicht von nicht weniger als ungefähr 1000 aufweist.

8. Verfahren nach irgend einem der vorangehenden Ansprüche, wobei die Beschichtung als Doppelschicht ausgebildet wird, die eine innere Lage, enthaltend ein hydrophiles Material, und eine äussere Lage, enthaltend eine hydrophobe Lage, beinhaltet.

9. Verfahren nach irgend einem der vorangehenden Ansprüche, wobei der Wirkstoff eine Cyclodextrin-Einschlussverbindung des Hydrochlorids des 4-Guanidino-methylcyclohexancarbonsäure-(2'-benzyloxycarbonyl)phenylesters ist.

**Revendications**

**Revendications pour les Etats contractants suivants : AT, BE, CH, DE, DK, FR, GB, IT, LI, LU, NL, SE**

1. Composition enrobée, administrable par la voie orale, comprenant une substance pharmaceutiquement active, stable à la chaleur, qui comprend des granules comprenant de fines particules de ladite substance active adhérant les unes aux autres à l'aide d'une première matière thermofusible servant de liant, chacun desdits granules étant enrobés d'une seconde matière thermofusible possédant un point de fusion inférieur à celui de la première matière thermofusible en une proportion de 0,1 à 2 parties en poids par partie en poids du granule en question, lesdits granules étant préparés par un processus de granulation à force centrifuge sans utilisation d'un quelconque solvant.

2. Composition enrobée suivant la revendication 1, caractérisée en ce que les granules ne libèrent sensiblement pas ladite substance pharmaceutiquement active avant l'écoulement d'un délai d'au moins 5 minutes après l'administration par la voie orale.

3. Composition enrobée suivant la revendication 1 ou la revendication 2, caractérisée en ce que la première matière thermofusible est du polyéthylèneglycol qui peut comprendre une faible proportion d'alpha-d-tocophérol.

4. Composition enrobée suivant la revendication 3, caractérisée en ce que le polyéthylèneglycol possède un poids moléculaire moyen non inférieur à environ 1000.

5. Composition enrobée suivant l'une quelconque des revendications précédentes, caractérisée en ce que l'enrobage est formé en une bi-couche, qui comprend une couche interne comprenant une matière hydrophile et une couche externe comprenant une matière hydrophobe.

6. Composition enrobée suivant l'une quelconque des revendications précédentes, caractérisée en ce que la substance active est un composé à inclusion de cyclodextrine de chlorhydrate de 4-guanidinométhylcyclohexanecarboxylate de 2'-benzyloxycarbonylphényle.

7. Procédé de préparation d'une composition enrobée suivant l'une quelconque des revendications précédentes, caractérisé en ce que l'on granule par centrifugation un mélange comprenant ladite substance active et une première matière thermofusible à titre de liant, sous chauffage, sans l'emploi d'un quelconque solvant et on enrobe les granules ainsi obtenus d'une seconde matière thermofusible servant de matière d'enrobage, sous chauffage, le point de fusion de la seconde matière thermofusible étant inférieur à celui de la première matière thermofusible.

8. Procédé suivant la revendication 7, caractérisé en ce que l'on entreprend la granulation en faisant tourner le mélange dans des conditions telles que la première matière thermofusible soit maintenue à l'état fondu.

9. Procédé suivant la revendication 7 ou la revendication 8, caractérisé en ce que l'on entreprend l'enrobage en faisant tourner les granules tout en leur appliquant la seconde matière thermofusible dans des conditions telles que la seconde matière thermofusible soit maintenue à l'état fondu.

10. Procédé suivant l'une quelconque des revendications 7 à 9, caractérisé en ce que la seconde matière thermofusible est un mélange comprenant une matière hydrophile et une matière hydrophobe en proportions de 5 % à 30 % en poids et de 10 % à 60 % en poids respectivement, sur base du mélange.

**Revendications pour les Etats contractants suivants : ES, GR**

1. Procédé de préparation d'une composition enrobée, administrable par la voie orale, comprenant une substance pharmaceutiquement active, stable à la chaleur, qui comprend des granules comprenant de fines particules de ladite substance active adhérant les unes aux autres à l'aide d'une première matière thermofusible servant de liant, chacun desdits granules étant enrobés d'une seconde matière thermofusible possédant un point de fusion inférieur à celui de la première matière thermofusible en une proportion de 0,1 à 2 parties en poids par partie en poids du granule en question, lequel procédé se caractérise en ce que l'on granule par centrifugation un mélange comprenant ladite substance active et une première matière thermofusible à titre de liant, sous chauffage, sans l'emploi d'un quelconque solvant et on enrobe les granules ainsi obtenus d'une seconde matière thermofusible servant de matière d'enrobage, sous chauffage.

2. Procédé suivant la revendication 1, caractérisé en ce que l'on entreprend la granulation en faisant tourner le mélange dans des conditions telles que la première matière thermofusible soit maintenue à l'état fondu.

3. Procédé suivant la revendication 1 ou la revendication 2, caractérisé en ce que l'on entreprend l'enrobage en faisant tourner les granules tout en leur appliquant la seconde matière thermofusible dans des conditions telles que la seconde matière thermofusible soit maintenue à l'état fondu.

4. Procédé suivant l'une quelconque des revendications précédentes, caractérisé en ce que la seconde matière thermofusible est un mélange comprenant une matière hydrophile et une matière hydrophobe en proportions de 5 % à 30 % en poids et de 10 % à 60 % en poids respectivement, sur base du mélange.

5. Procédé suivant l'une quelconque des revendications précédentes, caractérisé en ce que les granules ne libèrent sensiblement pas ladite substance pharmaceutiquement active avant l'écoulement d'un délai d'au moins 5 minutes après l'administration par la voie orale.

6. Procédé suivant l'une quelconque des revendications précédentes, caractérisé en ce que la première matière thermofusible est du polyéthylèneglycol qui peut comprendre une faible proportion d'alpha-d-tocophérol.

7. Procédé suivant la revendication 6, caractérisé en ce que le polyéthylèneglycol possède un poids moléculaire moyen non inférieur à environ 1000.

8. Procédé suivant l'une quelconque des revendications précédentes, caractérisé en ce que l'enrobage est formé en une bi-couche, qui comprend une couche interne comprenant une matière hydrophile et une couche externe comprenant une matière hydrophobe.

9. Procédé suivant l'une quelconque des revendications précédentes, caractérisé en ce que la substance active est un composé à inclusion de cyclodextrine de chlorhydrate de 4-guanidinométhylcyclohexanecarboxylate de 2'-benzyloxycarbonylphényle.

## Fig. 1

Graph: Dissolution (%) vs Time (min)

○ The 1st test solution
● Purified water

*Fig. 2*

○   The 1st test solution
●   Purified water

*Fig. 3*

○  The 1st test solution
△  The 2nd test solution
●  Purified water